# EUROPEAN PATENT SPECIFICATION

(11) **EP 0 911 015 B1**
(45) Date of publication and mention of the grant of the patent: **19.05.2004**
(21) Application number: 97830547.2
(22) Date of filing: 27.10.1997
(51) Int. Cl.: A61H 3/00, A61F 5/01

(54) **Orthopedic rehabilitation apparatus using virtual reality units**
Orthopädische Vorrichtung zur Rehabilitation mittels virtueller Realitätseinheiten
Appareil orthopédique de réhabilitation utilisant des dispositifs de réalité virtuelle

(43) Date of publication of application: 28.04.1999
(73) Proprietor: Ferrati, Benito, 20026 Novate Milanese (MI) (IT)
(72) Inventor: Ferrati, Benito, 20026 Novate Milanese (MI) (IT)
(74) Representative: Arena, Giovanni

(56) References cited:
- EP-A- 0 782 843
- DE-U- 29 511 509
- US-A- 3 204 954
- US-A- 4 256 098
- US-A- 5 282 460
- US-A- 5 562 572

## Description

The present invention relates to an orthopedic apparatus for walking and rehabilitating tetraplegic persons and for facilitating and stimulating the revival of comatose patients.

Orthopedic apparatus have been conceived for rehabilitating persons that are disabled in the motor abilities of their lower limbs (paraplegics, etc.) which are designed to allow walking for the purpose not only of improving the muscle, nerve and bone tonus, but also, and mainly, to stimulate and aid in the recovery of their motor ability.

Known orthopedic apparatus that are designed to stimulate and exercise the movement of the lower limbs and to allow walking, that have been designed by the present inventor include those disclosed in IT-B-1,153,225, and IT-B-1,178,548, as well as the Italian Utility Model No. 226,083 and EP-A-0782843.

In particular EP-A-0 782 843 discloses an orthopedic apparatus for walking and rehabilitation of a tetraplegics persons, presenting the following features:
- an exoskeleton for supporting the patient's body, articulated opposite his articulations, equipped with microcylinders and designed to move the jointed parts of the exoskeleton in accordance with the movements of the human gait;
- a programmed control unit to control the operation of said microcylinders such as to impress on the exoskeleton the movements of the human gait;
- a remote control for operation of the above mentioned control unit to allow the person to transmit commands to the unit for starting or stopping the lower limbs or adjusting the step speed;
- an electronic virtual reality unit designed to transmit to the person through a special helmet virtual reality pictures and stimulation interactive with the walking movement impressed by the exoskeleton on the person's body; and
- a framework with grips to be hold by the user for steading and supporting the same.

Other orthopedic apparatus are disclosed in US-A-3204954 AND US-A-4256098. These documents relate to track-supported harness means conceived to consent, without the assistance and presence of any assistant, physiotherapy walking exercises to paraplegics patients who have not completely lost their walking capability.

The object according to the present invention is to further improve the apparatus of the prior art, and more particularly, to strengthen the stimulation of the recovery of motor abilities in patients without motor capabilities of their lower and upper limbs, and to facilitate and stimulate the revival of comatose patients. Said object is achieved by an orthopedic apparatus comprising:
- an exoskeleton adapted to support a user's body, articulated at articulation positions of the same and provided with small actuators ("microcylinders") operated by compressed air, hydraulically or electrically, designed to move the articulated parts of the exoskeleton in accordance with the human gait;
- a programmed control unit for controlling the operation of said actuators to move the exoskeleton in accordance with the human gait;
- a remote control unit for controlling said programmed control unit with commands for stopping, starting or controlling the speed of the human gait, provided for being always in the hands of an attendant in order to ensure a timely operation of the same during any phase of the walking;
- an electronic virtual reality unit for transmitting to the user, by means of a virtual reality helmet, suitable virtual reality pictures and stimulation interactive with the human gait that is impressed on the user by the exoskeleton;
- a rail suspended at a distance from the ground, for supporting and guiding a ball bearing or a slide slidable inside or on said rail;
- a metal framework supported by said ball bearing or slide and provided with two suspenders or rods for supporting the exoskeleton of the patient.

Further objects, features and advantages of the present invention will be apparent from the following detailed description taken in conjunction with the accompanying drawings, in which:
- Fig.1: illustrates a person using a walking apparatus of a known type;
- Fig.2: illustrates an example of an apparatus according to the present invention.

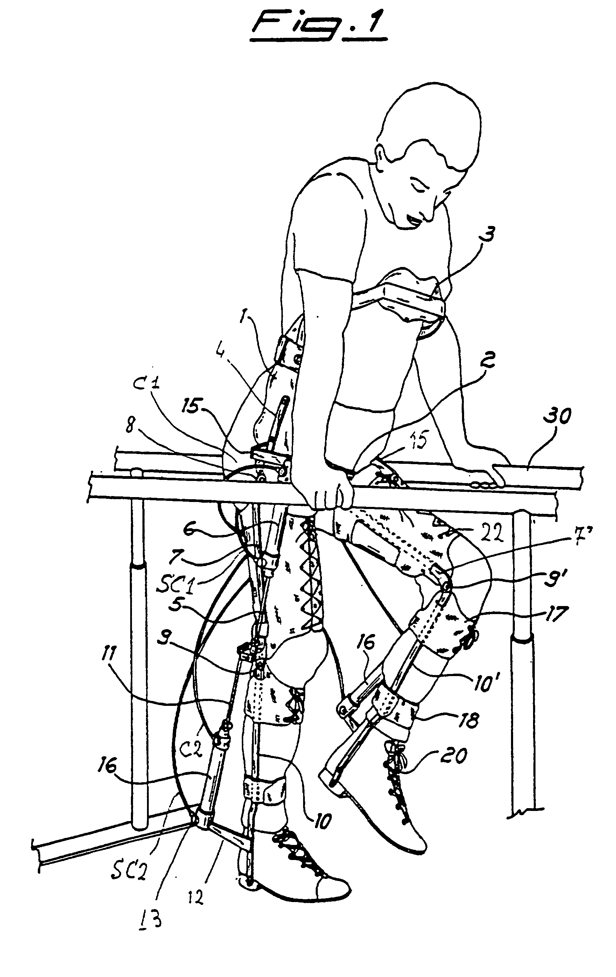

Fig.1 illustrates a disabled person who is leaning with his hands on two fixed bars. The person is equipped with a walking apparatus of the type which was described above and is disclosed in Italian patent No. 1,178,548. This apparatus comprises a metal corset 1 that is attached to the chest and the pelvis by two attachment devices 2 and 3. On two sides of the corset are fixed metal rods 4 that each have an integral extension 15. At the lower end of the rod 4, at a hinge point 8, there is attached a rod 7. The rod 7 extends along the external side of the thigh. A corresponding rod 7' is arranged parallel on the internal side of the thigh, and together with the rod 7 forms a framework of the thigh. A cylinder 6 operated by compressed air or hydraulically is hingedly connected to an end of the extension 15 at an upper end thereof. The cylinder 6 has a rod 5 having a lower end that is connected through a joint to a lower end of the rod 7.

The rod 7 has a lower end that terminates at a hinge 9 that connects the rod 7 with a lower rod 10. The lower rod 10 is part of the leg framework. On the lower part of the rod 10 there is fixed an integral extension 12. The extension 12 is shown enlarged in the drawing in order to provide greater clarity.

A cylinder 16 is connected and hinged to an end of the extension 12, and has a rod 11 that is connected through a joint to a small bracket that is fixed to the lower end of the rod 7. There is a corresponding rod 10' similarly hinged together with the rod 7' for the inner side of the leg.

A plurality of laced bands 17, 18 and 22 allow the rods 7 and 7' and 10 and 10' to be tightened to the thighs and legs of the patient.

Extension and retraction of the rod 5 of the cylinder 6 causes relative movement between the thigh and the pelvis. Extension and retraction of the rod 11 causes relative movement between the thigh and the lower leg. The cylinders 6 and 16, which are microcylinders, are operated by tubes C1, C2, SC1 and SC2, which provide for the inlet and outlet of a working fluid such as compressed air or hydraulic fluid. The patient wears on his back a pack (not illustrated) in which are housed compressed air bottles, for example, for operating the cylinders.

Solenoid valves are provided for operating the microcylinders. They are controlled by an electronically or hydraulically operated control unit that is fixed to the patient's belt, and appropriately programmed in order to move the lower jointed parts of the device in accordance with the human gait. The control unit is in turn controlled by means of a manually operated remote control which is located on the patient's corset or belt, and that permits the patient to transmit to the control unit commands for starting or stopping the movement of the lower parts or for adjusting the step speed.

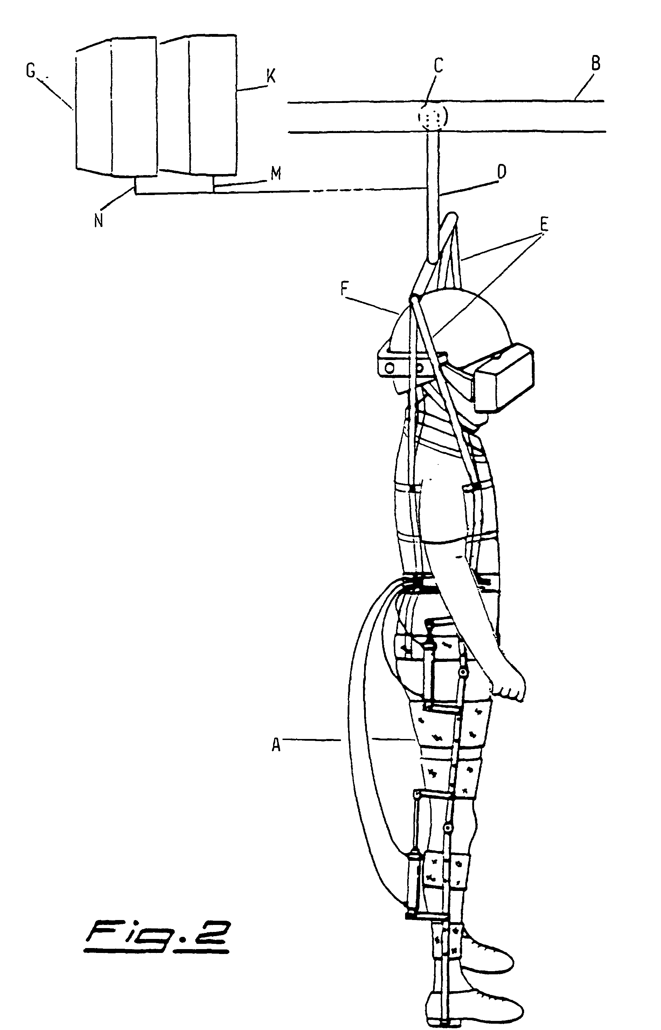

Fig.2 illustrates a disabled person employing a rehabilitation apparatus in accordance with an embodiment of the present invention.

This apparatus comprises:
- a sling (exoskeleton) A laced to patient's body, similar to that illustrated in figure 1 but improved over the sling of figure 1 as will be explained below;
- a rail B suspended at a distance from the ground higher than human height, for supporting and guiding a ball bearing or a slide C slidable inside or on said rail;
- a metal framework D supported by said ball bearing or slide C and provided with two suspenders or roads E for supporting the sling of the patient;
- a helmet F worn by the patient, provided with ear-phones and viewers that are connected to an electronic virtual reality unit G.

The exoskeleton that is shown in Fig. 2 is improved with respect to that of Fig. 1 by making the frame members that were previously metallic parts out of an ultralight material such as a carbon fiber and titanium, in order to reduce the weight on the patient's body in movement. Moreover The sling is extended on the upper part in order to support the patient's bust and, in case, even his head or the helmet F.

The rail B covers a circular route. Nevertheless it could cover a rectilinear route. In the last case a device should be provided which might cause the framework

D to revolve 180 degree at the end of the run so that even the patient, with the help of a hospital attendant, might revolve and begin a new run in the opposite direction.

As refers the remote control unit for controlling the programmed control unit which controls the operation of the exoskeleton actuators, it shall always be in the hands of an hospital attendant in order to ensure a timely operation of the same during any phase of the walking.

As refers the means supplying pressurized fluid it can be located in a special delivery unit K whose delivering duct M can have a passage extending through the framework D, or at least an anchoring point on the same framework.

Said supplying means can be either a simple compressed gas (for example air) bottle, or a compressor, or a pump that provides hydraulic operation of the microcylinders. The special delivery unit K and its delivery duct M, whatever its configuration, of course has a fluid passage or connection to the distribution box for operation of the microcylinders.

Even the cable N that connects the electronic virtual reality unit G to the patient's helmet can pass through the framework D, or at least have an appropriate anchor point on the same. The virtual reality unit G is programmed to transmit to the patient virtual reality pictures and stimulation that is interactive with the walking movement that is impressed by the exoskeleton on the patient's body. This involves the patient emotionally, which is important, because therapeutic efficacy is proportional to the emotional involvement of the patient. Examples of the types of virtual reality pictures and stimulation are pictures and stimulation that are related to the path which, according to the pictures projected in the helmet, the patient believes he is following, and can reflect situations of emergency and danger that occur along the path, or pictures and stimulation that are related to paths that are particularly dear or at least significant in the memory of the patient. Sounds and voices that can be either danger signals, or sounds, music and voices that are particularly dear to the patient, can also be provided.

In order to explain the utility of the equipment in accordance with the present invention, and in particular the use of virtual reality with the present invention, the following observations are pertinent.

It is known that lesions or amputations of the spinal medulla due to injuries to the spinal column, inflammation of the medulla or the vertebra, tumors or serious vascular disorders can cause paralysis of the lower limbs due to the fact that the lesions constitute a break in the transmission of nervous motor impulses from the brain. It is also known that the motor program of the limbs is not located only in the brain, but also in the spinal medulla, as proven by the fact that animals run on their own legs for a few moments even after amputation of the head. The above observations serve as a key for the interpretation of rehabilitation methods that are based on a program of exercise for the patient in walking in a manner as natural as possible with the aid of special support and motor mechanisms for the legs. These methods allow not only the reactivation or rememorization of the motor program of the legs in the spinal medulla, but also progressively reactivate the passage of the nervous impulses from the brain in the injured zone of the medulla. The usefulness of virtual reality in this situation is based on the properties of adrenaline and noradrenaline and on the fact that the secretion of adrenaline is stimulated upon the occurrence of emergency situations such as strenuous efforts, emotions, danger signals, arterial hypotension, asphyxia, hypothermia, etc. The growth of the concentration of adrenaline in the blood can be up to levels that are 300 times normal.

The adrenaline and noradrenaline hormones have the function of preparing the system both physically and emotionally to face emergency situations.

Among their various effects is a stimulation of the nervous system.

In particular, adrenaline and noradrenaline act as neurotransmitters. That is, they are chemical intermediaries that allow the transmission of nervous impulses from one neuron to another.

It is thus understandable how useful virtual reality could be during a patient's walking exercises as a means of stimulating the transmission of nervous impulses in the injured zone of the medulla through virtual reality pictures, sensations and emotions that are designed to stimulate the increase of the adrenaline levels in the blood.

Therefore the voices, sounds and pictures transmitted by the virtual reality helmet prove very helpful in facilitating and stimulating the revival of comatose patients. It could be advantageous to use the apparatus according to the present invention without the use of the virtual reality helmet, either before starting therapy with the helmet, in order to allow the patient to gain familiarity and practice in the use of the conveyor belt, or after the therapy cycle with the helmet, when the voluntary mobility of the lower limbs has begun to appear.

## Claims

1. An orthopedic apparatus for walking and rehabilitating persons without motor capabilities of their lower and upper limbs, and for facilitating and stimulating the revival of comatose patients, comprising:
- an exoskeleton (A) adapted to support a user's body, articulated at articulation positions of the same and provided with small actuators ("microcylinders") operated by compressed air, hydraulically or electrically, designed to move the articulated parts of the exoskeleton in accordance with the human gait;
- a programmed control unit for controlling the operation of said actuators to move the exoskeleton in accordance with the human gait;
- a remote control unit for controlling said programmed control unit with commands for stopping, starting or controlling the speed of the human gait, provided for being always in the hands of an attendant in order to ensure a timely operation of the same during any phase of the walking;
- an electronic virtual reality unit (G) for transmitting to the user, by means of a virtual reality helmet, suitable virtual reality pictures and stimulation interactive with the human gait that is impressed on the user by the exoskeleton; **characterized by** the fact that the apparatur further comprises:
- a rail (B) suspended at a distance from the ground, for supporting and guiding a ball bearing or a slide (C) slidable inside or on said rail;
- a metal framework (D) supported by said ball bearing or slide and provided with two suspenders or rods (E) for supporting the exoskeleton of the patient; and
- a device which causes the framework (D) to revolve 180 degree at the end of the run so that even the patient, with the help of the attendant, might revolve and begin a new run in the opposite direction.

## Patentansprüche

1. Orthopädisches Gerät zum Laufen und Rehabilitieren von Personen ohne motorische Fähigkeiten ihrer unteren und oberen Gliedmaßen und zur Erleichterung und Stimulierung der Wiederbelebung von Komapatienten, mit:
- einem Stützschienenkorsett (A), welches den Körper des Benutzers abstützen kann und an dessen Gelenkpunkten gelenkig ist und mit kleinen Aktuatoren (Mikrozylindern) versehen ist, welche durch Druckluft, hydraulisch oder elektrisch betätigt werden und die gelenkigen Teile des Stützschienenkorsetts entsprechend dem menschlichen Gang bewegen können;
- einer programmierten Steuereinheit zum Steuern des Betriebs der Aktuatoren derart, dass sie das Stützschienenkorsett entsprechend dem menschlichen Gang bewegen;
- einer Fernsteuereinheit zum Steuern der programmierten Steuereinheit mit Befehlen zum Anhalten, Starten oder Steuern der Geschwindigkeit des menschlichen Gangs, wobei die Fernsteuereinheit dazu bestimmt ist, sich immer in den Händen eines Beaufsichtigenden zu befinden, um in jeder Phase des Gehens einen zeitlich richtigen Betrieb des Gerätes sicherzustellen;
- eine elektronische Virtuelle-Realität-Einheit (G), welche an den Benutzer mit Hilfe eines Virtuelle-Realität-Helm geeignete Virtuelle-Realität-Bilder und Stimulationen interaktiv mit dem menschlichen Gang überträgt, der dem Benutzer von dem Stützschienenkorsett aufgeprägt wird;
**dadurch gekennzeichnet, dass** das Gerät weiterhin aufweist:
- eine in einem Abstand vom Boden aufgehängte Schiene (B) zum Tragen und Führen eines Wälzlagers oder Schlittens (C), der innerhalb der Schiene gleiten kann;
- einen Metallstützrahmen (D), der von dem Wälzlager oder dem Schlitten getragen wird und mit zwei Aufhängungen oder Stangen (E) zum Tragen des Stützschienenkorsetts des Patienten versehen ist; und
- eine Vorrichtung, welche den Stützrahmen (D) veranlasst, am Ende des Laufweges eine Umkehrung von 180° durchzuführen, so dass der Patient, mit Hilfe des Beaufsichtigenden, umkehren und einen neuen Weg in entgegensetzter Richtung beginnen kann.

## Revendications

1. Appareil orthopédique pour faire marcher et réhabiliter des personnes sans capacité motrice de leurs membres inférieurs et supérieurs, et pour faciliter et stimuler la récupération de patients comateux, comprenant :
- un exosquelette (A) adapté à porter le corps d'un utilisateur, articulé à des positions d'articulation de celui-ci et muni de petits actionneurs ("microcylindres") actionnés à l'air comprimé, hydrauliquement ou électriquement, prévus pour déplacer les parties articulées de l'exosquelette en accord avec la démarche humaine ;
- un module de télécommande pour commander le module de commande programmé par des commandes d' arrêt, de marche, et de contrôle de vitesse de démarche, prévu pour être toujours dans les mains d'un assistant pour assurer un fonctionnement adapté pendant toutes les phases de la marche ;
- un module électronique à réalité virtuelle (G) pour transmettre à l'utilisateur, au moyen d'un casque à réalité virtuelle, des images appropriées de réalité virtuelle et de stimulation interactive avec la démarche humaine qui est imposée à l'utilisateur par l'exosquelette,
**caractérisé en ce que** cet appareil comprend en outre :
- un rail (B) suspendu à une certaine distance du sol pour porter et guider un roulement ou un coulisseau (C) pouvant coulisser dans ou sur le rail ;
- un châssis métallique (D) porté par le roulement ou le coulisseau et muni de deux suspensions ou cordes (E) pour porter l'exosquelette du patient ; et
- un dispositif qui amène le cadre (D) à tourner de 180° à la fin du parcours de sorte que même le patient, avec l'aide de l'assistant, puisse tourner et commencer une nouvelle marche en direction opposée.
